Europäisches Patentamt

European Patent Office

Office européen des brevets

(11) Veröffentlichungsnummer: **0 362 244 B1**

## EUROPÄISCHE PATENTSCHRIFT

(45) Veröffentlichungstag der Patentschrift: **06.04.94**

(51) Int. Cl.5: **C12N 15/82**, C12N 15/87

(21) Anmeldenummer: **88904525.8**

(22) Anmeldetag: **20.05.88**

(86) Internationale Anmeldenummer:
**PCT/EP88/00452**

(87) Internationale Veröffentlichungsnummer:
**WO 88/09374 (01.12.88 88/26)**

(54) **EMBRYONEN VON NUTZPFLANZEN ALS AUFNAHMESYSTEM FÜR FREMDE GENETISCHE INFORMATION.**

(30) Priorität: **22.05.87 DE 3717301**

(43) Veröffentlichungstag der Anmeldung:
**11.04.90 Patentblatt 90/15**

(45) Bekanntmachung des Hinweises auf die Patenterteilung:
**06.04.94 Patentblatt 94/14**

(84) Benannte Vertragsstaaten:
**AT BE CH DE FR GB IT LI LU NL SE**

(56) Entgegenhaltungen:
**EP-A- 0 164 575**
**EP-A- 0 241 963**
**EP-A- 0 267 159**
**WO-A-83/01176**

**Chemical Abstracts, vol. 86, 1977 (Columbus, Ohio, US), D. Hess, p. 206, abstract no. 13946p**

(73) Patentinhaber: **Max-Planck-Gesellschaft zur Förderung der Wissenschaften e.V.**
**Bunsenstrasse 10**
**D-37073 Göttingen(DE)**

(72) Erfinder: **TÖPFER, Reinhard**
**Max-Planck-Institut für Züchtungsforschung**
**Egelspfad D-5000 Köln 30(DE)**
Erfinder: **SCHELL, Josef, S.**
**Max-Planck-Institut für Züchtungsforschung**
**Egelspfad D-5000 Köln 30(DE)**
Erfinder: **STEINBISS, Hans-Henning**
**Max-Planck-Institut für Züchtungsforschung**
**Egelspfad D-5000 Köln 30(DE)**

(74) Vertreter: **VOSSIUS & PARTNER**
**Postfach 86 07 67**
**D-81634 München (DE)**

J. Mol. Biol., vol. 43, 1969, L. Ledoux et al., pp. 243-262

Naturwissenschaften, vol. 59, 1972, Springer, D. Hess, pp. 348-355

Science, vol. 237, 1987, J St. Schell, pp. 1176-1183

Z. Pflanzenphysiol., vol. 61, 1969, D. Hess, pp. 286-298

Plant Molecular Biology, vol. 7, no. 1, 1986, Martinus Nijhoff Publishers (Dordrecht, NL), A.C.F. Graves et al., pp. 43-50

Methods in Enzymology, vol. 101, 1983, Academic Press, Inc., G.-Y. Zhou et al., pp. 433-481

Nature, vol. 249, 3 May 1974, L. Ledoux et al., pp. 17-21

Theoretical and Applied Genetics,vol. 58, 1980, Springer, V.N. Soyfer, pp. 225-235

Chemical Abstracts, vol. 76, 13 March 1972, (Columbus, Ohio, US), L. Ledoux et al., p. 68, abstract no. 54778x

Chemical Abstracts, vol. 90, 1979, 21 May 1979, (Columbus, Ohio, US), P. Tichy et al., p. 290, abstract no. 164890z

Z. Pflanzenphysiol., vol. 94, 1979, J. Korohoda et al., pages 95-99

**Beschreibung**

Die Erfindung betrifft mechanisch freigelegte reife Embryonen von Nutzpflanzen mit einem Wassergehalt von höchstens etwa 20 Gewichtsprozent als Aufnahmesystem für fremde genetische Information in Form von DNA oder RNA erhältlich durch Abschleifen der Samenschale und Verletzung des Embryos. Ferner betrifft die Erfindung mechanisch freigelegte reife Embryonen Von Nutzpflanzen mit einem Gehalt an eingeschleuster fremder genetischer Information, Verfahren zur Herstellung der genannten Embryonen und ihre Verwendung zur Regenerierung und Züchtung von transgenen Nutzpflanzen.

Agrobacterium tumefaciens als gut untersuchtes und einfach zu handhabendes Gentransfersystem, in der Hauptsache für dicotyle Pflanzen, ist bisher auf nur wenige monocotyle Pflanzen angepaßt worden. Ein direkter Gentransfer auf Pflanzen, d.h. ein Gentransfer ohne bakterielle oder virale Vektoren ist daher eine alternative Transformationsmethode und könnte für Pflanzen, die schlechte Wirte für Agrobacterium darstellen, zu größerer Bedeutung gelangen. Gerade zu diesen Pflanzen zählen viele wirtschaftlich bedeutsame und gentechnologisch bisher kaum handhabbare Nutzpflanzen, insbesondere die Getreidepflanzen und Leguminosen.

Etabliert ist der Einsatz von Protoplasten als Objekt für den direkten Gentransfer. Dies gilt auch für Protoplasten von Getreidepflanzen. Bisher ist es aber nur in Ausnahmefällen gelungen, Getreideprotoplasten zu reifen Pflanzen zu regenerieren. Deshalb muß weiter nach alternativen Wegen gesucht werden.

Mit der Arbeit von De la Pena et al., Nature 325 (1987) 274-276, ist ein neuer Weg des direkten Gentransfers auf eine Nutzpflanze erstmals erfolgreich eingeschlagen worden. Plasmid-DNA wurde in junge Roggenähren etwa 2 Wochen vor der Meiose injiziert. In der Fl-Generation wurden transformierte Pflanzen selektiert. Außer dieser Methode wurden auch Methoden beschrieben, denen die Inkubation verschiedener Explantate mit nackter DNA gemeinsam ist.

EP-A1 241 963 beschreibt als Aufnahmesystem für fremde DNA dienendes pflanzliches Material.

Die Behandlung von Pollen, Samen, Keimlingen oder multizellulären Strukturen höherer Pflanzen mit fremder DNA ist bereits bekannt, wird aber wegen des Fehlens eingehender Beweise kontrovers diskutiert.

Der Erfindung liegt somit die Aufgabe zugrunde, ein neues System zum Einschleusen fremder genetischer Information in Nutzpflanzen, insbesondere in Getreidepflanzen und in Leguminosen, bereitzustellen.

Die Lösung dieser Aufgabe wird durch die Bereitstellung der in den Patentansprüchen gekennzeichneten Ausführungsformen erzielt.

Gegenstand der Erfindung sind somit mechanisch freigelegte reife Embryonen von Nutzpflanzen mit einem Wassergehalt von höchstens etwa 20 Gewichtsprozent als Aufnahmesystem für fremde genetische Information in Form von DNA oder RNA, die durch Abschleifen der Samenschale und Verletzung des Embryos erhältlich sind. Embryonen reifer Samen sind in der Lage während der Quellung, im isolierten Zustand oder im Samen (beispielsweise mechanisch freigelegt), fremde genetische Information aufzunehmen. Diese "fremde genetische Information" kann sowohl DNA als auch RNA sein, die sich selbst repliziert und infektids ist oder stabil in das Genom integriert ist. In gleicher Weise werden Viroide oder Viren vom Begriff "fremde genetische Information" erfaßt. Als "fremde genetische Information" wird auch eine genetische Information bezeichnet, die natürlich in der Nutzpflanze vorkommt, jedoch zur Erzielung phänotypischer Veränderungen zusätzlich in weiteren Kopien in die Nutzpflanze eingebracht wird, beispielsweise können auf diesem Weg natürlich in der Nutzpflanze verkommende Gene amplifiziert werden. Neben Embryonen reifer Samen ist es aber auch möglich, in einen samenruheähnlichen Zustand überführte, somatische Embryonen oder künstliche Samen (artificial seeds) einzusetzen.

Gegenstand der Erfindung sind ferner die bezeichneten mechanisch freigelegten reifen Embryonen von Nutzpflanzen mit einem Gehalt an eingeschleuster fremder genetischer Information in Form von DNA oder RNA.

Die nachstehend verwendeten Begriffe "offengelegt", "mechanisch freigelegt" und "isoliert" sind hier folgendermaßen definiert:

Als "offengelegte Embryonen" werden Embryonen bezeichnet, deren Samenschale zumindest teilweise entfernt ist, so daß der Embryo für eine fremde genetische Information in Form von DNA oder RNA zugänglich wird. Der Begriff offengelegte Embyonen ist somit als Oberbegriff zu verstehen.

Mit dem Begriff "isolierte Embryonen" werden Embryonen bezeichnet, die vom Speichergewebe des Samens abgetrennt sind. Ein Verfahren zur Herstellung isolierter Weizenembryonen ist aus Johnston & Stern, Nature 179 (1957), Seiten 160 bis 161, bekannt. Johnston & Stern beschreiben jedoch nicht die Verwendung solcher Embryonen zur Regenerierung und Züchtung transgener Nutzpflanzen.

Mit dem Begriff "mechanisch freigelegte Embryonen" werden Embryonen bezeichnet, die sich noch im strukturellen Verband des Samens befinden und bei denen die Samenschale derart geöffnet ist, daß die fremde genetische Information in den Embryo gelangen kann. Die erfindungsgemäßen mechanisch freigelegten Embryonen gestatten bei der Regenerierung und Züchtung transgener Nutzpflanzen eine noch höhere Ausbeute an regenerierten Pflanzen als isolierte Embryonen.

Der Zusammenhang der Begriffe "offengelegte", "mechanisch freigelegte" und "isolierte" Embryonen kann also folgendermaßen verdeutlicht werden:

```
                    Offengelegte Embryonen
                 (Samenschale ist ganz-oder
                     teilweise entfernt)


Isolierte Embryonen                    Mechanisch freigelegte Embryonen
(Samenschale und Speichergewebe         (Samenschale ist nur
sind vollständig entfernt; vgl.          geöffnet bzw. teil-
Johnston und Stern)                      weise entfernt; erst-
                                         mals in dieser Erfin-
                                         dung beschrieben)
```

Erfindungsgemäß kann die fremde genetische Information entweder frei in den Zellen vorliegen, oder in das Genom der Zellen integriert sein. Eine in den Zellen der Embryonen frei vorliegende genetische Information könnte man auch als "gentechnologischen Pflanzenschutz" bezeichnen.

Offengelegte Embryonen können als Pforte für genetische Information dienen, die sich selbst vermehrt und in der Pflanze ausbreiten kann, mit dem Ziel auf die ganze aus dem Embryo entstehende Pflanze überzugreifen bzw. diese zu infizieren.

Embryonen können für einen begrenzten Zeitraum das Genprodukt fremder genetischer Information herstellen, welches mit den heute üblichen Meßmethoden zu erfassen ist. Damit besteht eine Alternative zur Messung der transienten Expression chimärer Gene in den aufwendiger zu handhabenden Protoplasten. Es ist eine gewebespezifische Expression zu erwaten, da es sich um ein vollständiges gewehe handelt. Die Spezifität der Expression läßt sich ferner durch Verwendung bestimmter Promotoren steuern.

Die erfindungsgemäßen mechanisch freigelegten reifen Embryonen von Nutzpflanzen werden durch mechanische Behandlung des Samens erhalten. Durch das Entfernen z.B. durch Abschleifen der Samenschale im Bereich des Embryos, kann der Embryo freigelegt werden. Dabei erfolgt das Abschleifen der Samenschale derart, daß der Embryo für eine fremde genetische Information in Form von DNA oder RNA zugänglich wird, jedoch in seiner Keimfähigkeit nicht wesentlich beeinträchtigt wird. Das erfindungsgemäße Abschleifen der Samenschale ist schonend für den Embryo und rasch durchführbar. Daher wird es erfindungsgemäß ermöglicht, in kurzer Zeit zahlreiche Nutzpflanzen ausgehend von behandelten Embryonen zu züchten, die einen Gehalt an fremder genetischer Information aufweisen.

Die Erfindung betrifft auch ein Verfahren zur Herstellung von mechanisch freigelegten reifen Embryonen von Nutzpflanzen mit einem Gehalt an eingeschleuster fremder genetischer Information in Form von DNA oder RNA, bei dem man mechanisch freigelegte reife Embryonen von Nutzpflanzen mit einem Wassergehalt von höchstens etwa 20 Gewichtsprozent mit einer wäßrigen Lösung der genetischen Information in Form von DNA oder RNA behandelt und wobei die Embryonen durch Abschleifen der Samenschale und Verletzung des Embryos erhältlich sind.

In einer weiteren bevorzugten Ausführungsform des erfindungsgemäßen Verfahrens hat die wäßrige Lösung einen pH-Wert von 3 bis etwa 11.

Die erfindungsgemäßen mechanisch freigelegten reifen Embryonen von Nutzpflanzen sind hervorragend zur Regenerierung und Züchtung transgener Nutzpflanzen geeignet. Insbesondere sind sie zur Regenerierung von Getreidepflanzen und Leguminosen geeignet. Die Regenerierung und Züchtung der transgenen Nutzpflanzen ausgehend von den erfindungsgemäßen Embryonen kann in an sich bekannter Weise erfolgen.

Bei der Erarbeitung der erfindungsgemäßen Lösung wurden zunächst Experimente zur transienten Expression durchgeführt. Der Leitgedanke war dabei zunächst eine Expression bestimmter chimärer Gene für einige Tage (also transient) zu erreichen. Chimäre Gene sind in vitro zusammengefügte DNA-Bausteine, die ein funktionsfähiges künstliches Gen bilden und z.B. auf einem Plasmid lokalisiert sind. Die folgende Tabelle I zeigt darauf aufbauend die erfindungsgemäße Strategie am Beispiel des Weizens.

4

## Tabelle I

### Transiente Expression chimärer Gene in isolierten Weizenembryonen

Reproduzierbarkeit

Mechanismus der DNA-Aufnahme ⟷ Optimierung der DNA-Expression

Transformation

Nachfolgend werden experimentelle Ergebnisse beschrieben, angefangen von der Beobachtung einer gelegentlichen Expression eines chimären Gens, über die Suche nach reproduzierbaren Bedingungen, dem Versuch einer Klärung des Mechanismus der DNA-Aufnahme, verknüpft mit dem Streben nach einer Optimierung der Expression, bis hin zu Experimenten, aufbauend auf den gewonnenen Erkenntnissen, die zu transient oder stabil transformierten Pflanzen führen können. Das bedeutet, daß aus den behandelten Embryonen auch Pflanzen regeneriert werden können, die selbst oder deren Nachkommen transformiert sind, also fremde genetische Information enthalten, die gegebenenfalls in ihrem Genom integriert ist. Die Regeneration der behandelten Embryonen erfolgt nach üblichen Verfahren, nämlich beispielsweise durch Kultur auf gewöhnlichen Nährböden nach Kallusinduktion, Induktion somatischer Embryogenese oder unter Ausnutzung der natürlichen Nährstoffreserven des Samens.

Die in Tabelle I oben verwendeten Begriffe werden nachfolgend erläutert.

Reproduzierbarkeit

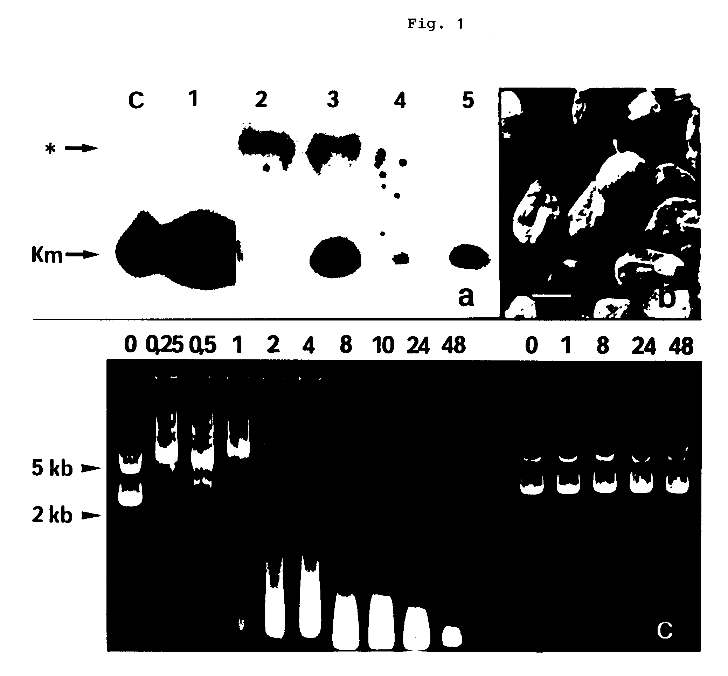

In den ersten Experimenten mit Weizenembryonen lagen die Hauptschwierigkeiten in der Reproduzierbarkeit. Zunächst waren mit 15 mg isolierten Embryonen gelegentlich NPT II Signale beobachtet worden. Erst die Erhöhung der Embryomenge auf 300 mg ergab reproduzierbare, wenn auch in der Intensität unterschiedliche NPT II Signale (Fig. 1a). Diese Schwankungen können als experimentelle Abweichungen angesehen werden, aber auch als eine verschieden starke DNA-Aufnahme und DNA-Expression einzelner Embryonen.

Abgesehen von der Tatsache, daß die NPT II Signale in drei identisch angelegten, aber unabhängig voneinander durchgeführten Experimenten gefunden wurden, müssen diese Daten doch kritisch betrachtet werden. Endogene Signale können ausgeschlossen werden, da in keinem Fall NPT II Aktivität in Extrakten von Embryonen ohne DNA-Behandlung gefunden wurden. Kontaminationen in Form von Bakterien oder Pilzen als Ursache für die beobachteten NPT II Signale können weitgehend ausgeschlossen werden. Gegen Bakterien wurde Claforan® dem Medium zugesetzt und Pilze waren während der Kultur nicht sichtbar. Traten dennoch Pilzkontaminationen im Laufe der Kultur auf, so wurden diese Teile verworfen. Weiterführende Versuche mit Fungiziden oder Sterilisierung der Embryonen, um Langzeitkulturen anzulegen, schlugen fehl. Statt dessen wurde auf eine Kultur von, angeschliffenen Samen ausgewichen. Weiterhin zeigten Kontrollexperimente mit Endospermstücken (berechnet auf gleiches Frischgewicht nach der Kultur) keine NPT II Aktivität.

## Mechanismus der DNA-Aufnahme

Nachdem die Reproduzierbarkeit erwiesen war, stellten sich die in enger Wechselwirkung stehenden Fragen nach dem Mechanismus der DNA-Aufnahme und der Optimierung der NPT II Expression. Zwei Barrieren muß die DNA überwinden, um zunächst einmal das Cytoplasma zu erreichen: Zellwand und Plasmalemma.

Die Wanderung von DNA-Molekülen durch Zellwände scheint unmöglich, es sei denn, der trockene Embryo weist gewisse Besonderheiten auf. Carpita et al., Science 205 (1979), 1144-1147, bestimmten die Porengröße in Zellwänden von Acer pseudoplatanus Suspensionskulturzellen mit 3,8 bis 4 nm. Das entspricht einer freien Permeabilität für Polyethylenglykol mit einem Molekulargewicht von 1300 bis 1600 und im Vergleich dazu für ein globuläres Protein bis zu 17000 d bei entsprechendem Molekülradius. Das aber dürfte viel zu klein sein für das Durchschlüpfen eines Plasmids von 4,15 kb ($2,7 \times 10^6$ d). Wieso kann aber dennoch transiente Expression in Embryonen beobachtet werden? Der Vergleich von Suspensionskulturzellen mit den "plasmareichen und zartwandigen Zellen" eines Embryos ist nur mit Einschränkungen erlaubt. So befinden sich Suspensionskulturzellen nicht in einem geordneten Zellverband und ihr Zellwandaufbau dürfte von dem der Embryonen verschieden sein. Zudem können die Quellungs-Vorgänge die Penetrierfähigkeit von Makromolekülen beeinflussen, was letztlich zu den oben angedeuteten Besonderheiten des trockenen Embryos zu zählen ist.

Ein anderer Weg der DNA durch die Zellwand könnte über Verletzungen des Embryos als Folge der Isolierung (Fig. 1b) oder des Anschleifens führen.

Das bedeutet, daß nur an verletzte Stellen grenzende Zellen fremde DNA aufnehmen können und für die Expression verantwortlich sind, es sei denn, es erfolgt eine Wanderung der DNA-Moleküle von Zelle zu Zelle durch Plasmodesmen.

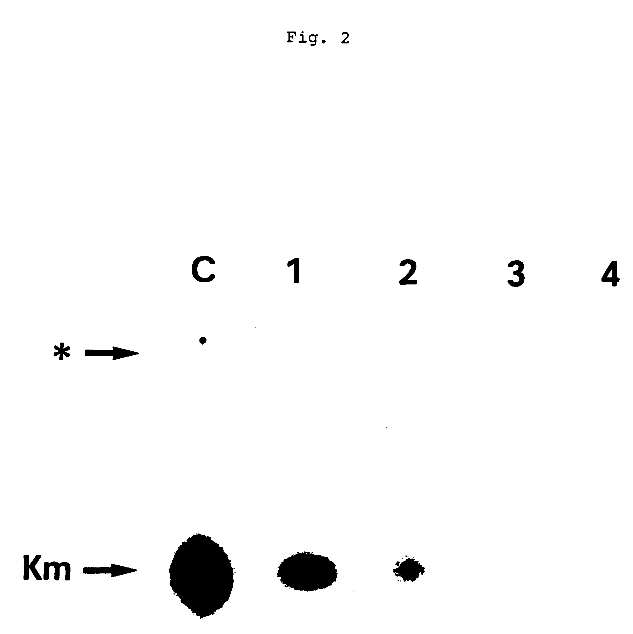

Die zarten Zellwände embryonaler Zellen während der Quellung oder die Verletzungen sind mögliche Ursachen die bestehende Barriere Zellwand zu passieren. Das Plasmalemma ist damit aber noch nicht überwunden. Es ist allgemein bekannt, daß DNA die Zellmembran nicht passieren kann. Chemische Agenzien, Elektropulse oder beides zusammen werden erfolgreich eingesetzt, um die Kontrollfunktion des Plasmalemma zu umgehen und transiente Expression in Protoplasten zu erreichen. Erfindungsgemäß (Fig. 1a und 2) wurde gezeigt, daß auch ohne solche Hilfsmittel eine transiente Expression vorhanden ist, aber beispielsweise mit DMSO verstärkt werden kann. Der trockene Embryo scheint daher über besondere Eigentümlichkeiten zu verfügen. Daß das frühe Stadium der Quellung kritisch ist, belegen sowohl Studien der Ultrastruktur, als auch Beobachtungen zur Permeabilität.

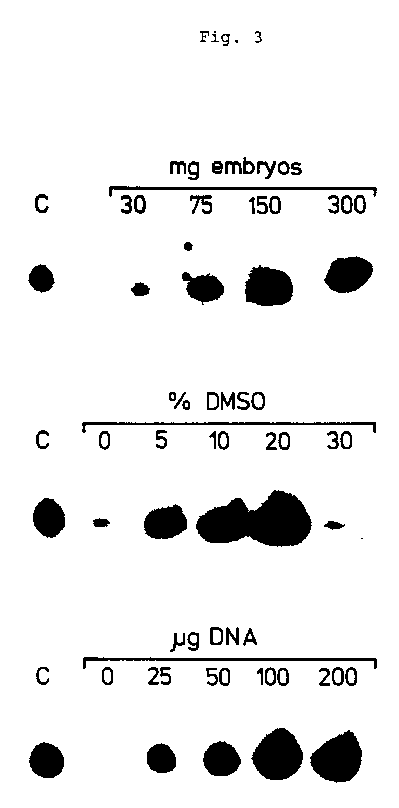

Eine DNA-Aufnahme, gemessen als NPT II Expression, erfolgt am besten in trockene Embryonen mit einem Wassergehalt von höchstens etwa 20 Gewichtsprozent. Je weiter die Quellung fortgeschritten ist, desto geringer fällt die Expression aus (Fig. 2). Dies steht in Einklang mit der möglichen Rekonstitution der Zellmembran. Ebenso entspricht der Einfluß von DMSO dieser Vorstellung, indem es die Membranrekonstitution oder allgemein die Membranpermeabilität beeinflussen könnte (Fig. 3).

## Optimierung der NPT II-Expression

Nach den Untersuchungen zum Mechanismus der DNA-Aufnahme, galt das Hauptinteresse der Optimierung der NPT II Expression, um mit den erreichten Verbesserungen Versuche zur stabilen Transformation unternehmen zu können.

Erfindungsgemäß wurde festgestellt, daß DMSO, bei Weizenembryonen im Optimum 20 %, einen fördernden Einfluß (Fig. 3, Mitte) auf, die NPT II Expression hat.

Zwei weitere Experimente führten zu verbesserten Bedingungen für transiente Expression. Anstatt 300 mg trockener Embryonen pro Versuch (Fig. 1a und 2) einzusetzen, konnten nach der Verwendung von DMSO zumindest gleiche Ergebnisse mit 150 mg Embryomaterial erzielt werden (Fig. 3 oben). Die Reproduzierbarkeit wurde sogar bis 30 mg Embryonen nachgewiesen, allerdings mit viel schwächeren Signalen. Eine Verbesserung brachte der Einsatz von 100 $\mu$g Plasmid DNA (Fig. 3, unten).

Die Figuren zeigen:

Figur 1:

(a) Ergebnis eines NPT II-Tests: C = Kontrolle, mit pRT100neo stabil transformierter Tabak-Kallus; Spur 1 = transient pRT100neo exprimierende Tabakprotoplasten; Spuren 2 bis 5 = keimende Weizenembryonen, Material von 300 mg gequollenen trockenen Embryonen, ohne DNA-Behandlung (Spur 2) und mit je 50 $\mu$g pRT100neo in 15 mM NaCL und 1,5 mM Trinatriumcitrat inkubiert (Spuren 3 bis 5); * = unspezifische pflanzliche Aktivität; Km = Kanamycinphosphat (Reaktionsprodukt der Neomycinphosphotransfe-

6

rase "NPT II").

(b) Isolierte, trockene Weizenembryonen der 0,71 mm Fraktion. Dieses Material wurde zur transienten Expression eingesetzt und besteht aus mehr oder weniger vollständigen Embryonen, Embryostücken und wenigen Endospermstücken. Die Pfeile weisen auf Verletzungen an den Embryonen hin.

(c) Abbau der Plasmid-DNA nach verschiedenen Inkubationszeiten (in Stunden) mit Weizenembryonen (links) und Stabilität der DNA bei Abwesenheit von Embryonen (rechts).

Figur 2: Ergebnis eines NPT II-Tests, durchgeführt mit keimenden Weizenembryonen (Doppelproben). Spuren 1 und 2 = Embryonen, die zunächst 30 Minuten mit DNA-Lösung (100 $\mu$g Plasmid DNA pro Milliliter, 15 mM NaCl, 1,5 mM Trinatriumcitrat), dann 30 Minuten mit 15 mM NaCl und 1,5 mM Trinatriumcitrat (ohne DNA) behandelt worden waren; Spuren 3 und 4 = Embryonen, die in umgekehrter Reihenfolge inkubiert wurden, zuerst mit Lösung ohne DNA, dann mit DNA-Lösung. C = positive Kontrolle; * = unspezifische pflanzliche Aktivität; Km = Kanamycinphosphat.

Figur 3: Ergebnisse von NPT II-Tests, durchgeführt mit keimenden Weizenembryonen: (oben) NPT II-Signale als Funktion der Embryomenge, unter Verwendung von 20 % DMSO und 50 $\mu$g DNA während der Inkubation; (Mitte) 150 mg Embryonen behandelt mit 50 $\mu$g DNA unter Zugabe von verschiedenen DMSO Konzentrationen; (unten) NPT II-Expression in Abhängigkeit von der Plasmid Konzentration unter Einsatz von 150 mg Embryonen und 20 % DMSO. C = positive Kontrolle; nur NPT II-Signale sind gezeigt.

Figur 4:

(a): Konstruktionsweg der Kassette pRT100. Neben den Pfeilen sind die zur Clonierung verwendeten, in den einzelnen Schemata nur die wichtigsten Restriktionsschnittstellen angegeben. Auffüllreaktionen von 5'- und Entfernen von 3'-überhängenden Enden erfolgten mit Klenow-Polymerase. Die intergenische Region des CaMV Isolats Cabb B-D wurde als EcoRI Subclon in fd 11-6 von Volker Matzeit (1982; Diplomarbeit an der Universität zu Köln) erhalten. Ausgehend von diesem Clon wurde das Poly-A Signal (schraffiert) als HphI-RsaI-Fragment in die HincII-Schnittstelle von pUC18 (Yanisch-Perron et al., Gene 33 (1985), 103-119) ligiert, der Promotor (punktiert) wurde als HphI-HincII Fragment in die HincII-Schnittstelle von pUC19 cloniert. Anschließend wurde er unter Umkehr der Orientierung mit seiner abgeschnittenen PstI-Schnittstelle in die aufgefüllte XhoI-Schnittstelle eines pUC19-Derivats, dessen SmaI- und KpnI-Schnittstellen durch XhoI- und NcoI-Schnittstellen ersetzt sind, umcloniert. Nach dem Deletieren der XbaI-Schnittstelle wurden Promotor und Poly-A Signal abschließend über ScaI und SstI zu pRT100 kombiniert.

(b) DNA Sequenz der Kassette in pRT100. Die Polylinkersequenzen sind durch Kleinbuchstaben gekennzeichnet, die CaMV-Sequenzen durch Großbuchstaben. Markiert sind die TATA Box (unterstrichen), der vermutliche Transkriptionsstart ($\mapsto$), das ATG in der NcoI Schnittstelle, der Polyadenylierungsbefehl (unterstrichen) und das vermutliche Transkriptionsende ($\dashv$).

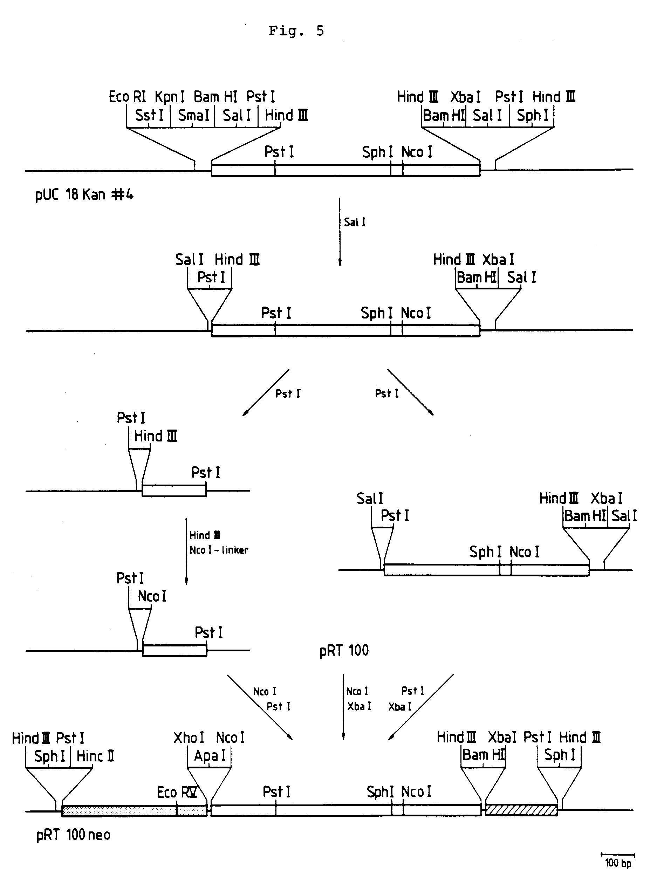

Figur 5: Schematische Übersicht der Konstruktion pRT100neo. Aus pUC18kan#4 wurde das NPT II-Gen als SalI-Fragment herausgeschnitten und in pUR250 (Rüther, Nucl. Acids Res. 10 (1982), 5765-5772) subcloniert. Ein etwa 180 bp großes PstI-Fragment mit dem 5'-Ende des NPT II-Gens wurde dann aus diesem Intermediat entfernt und in ein pUC18-Derivat ohne HindIII-Schnittstelle cloniert. In diesem Clon konnte nach HindIII-Spaltung, Auffüllreaktion und Ligation mit einem 8 bp großen Linker eine NcoI-Schnittstelle und damit ein ATG-Codon eingefügt werden. Aus letzterem Clon wurde abschließend das 5'-Ende des NPT II-Gens als NcoI-PstI-Fragment herausgeschnitten und zusammen mit dem 3'-Ende des NPT II-Gens als PstI-XbaI-Fragment in pRT100, geschnitten mit NcoI und XbaI, cloniert.

Material und Methoden:

Molekularbiologische Analysen wurden gemäß Maniatis et al. (1982) "Molecular Cloning, A Laboratory Manual", Cold Spring Harbor Laboratory Press, New York, durchgeführt.

Chimäre Gene

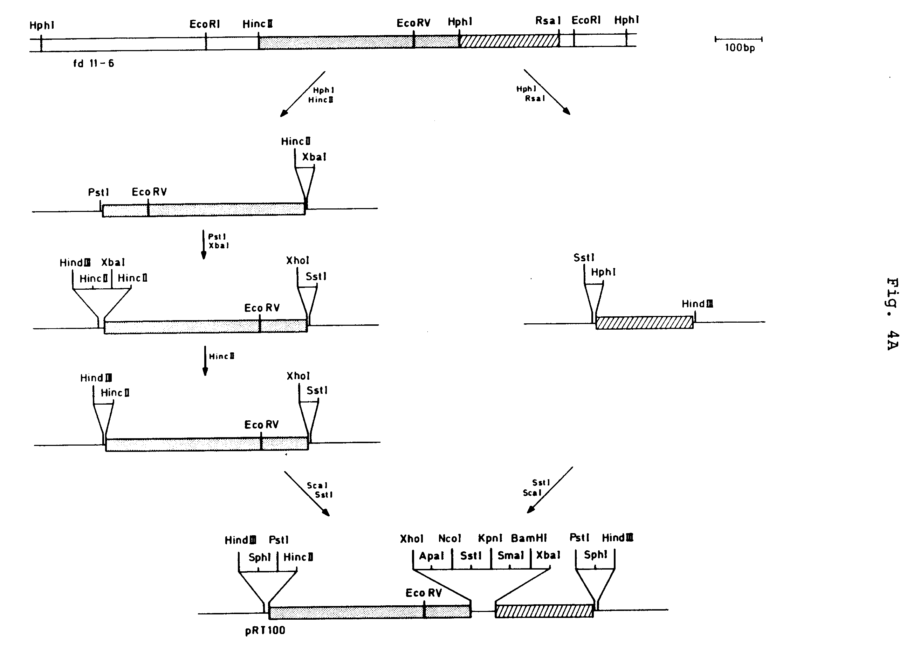

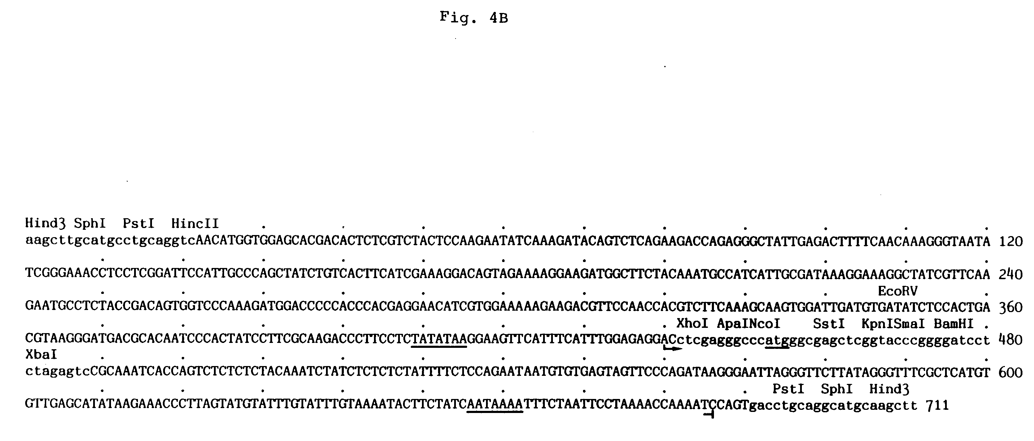

Erfindungsgemäß wurde als Modell das Plasmid pRT100neo mit dem vom Cauliflower Mosaik Virus (CaMV) 35S Promotor gesteuerten Neomycinphosphotransferase II-Gen (NPT II) als chimärem Marker-Gen verwendet. Die Konstruktion ist in den Figuren 4 und 5 detailliert beschrieben. Figur 4a veranschaulicht die Konstruktion des Expressionsvektors pRT100, der den 35S Promotor aus CaMV (Isolat Cabb B-D) und das zugehörige CaMV-Polyadenylierungssignal trägt. Figur 4b zeigt die Clonierungsschritte, die zu pRT100neo geführt haben. Es konnte gezeigt werden, daß die für transiente Studien eingesetzten Plasmide (fremde genetische Information) unabhängig von ihrer Konformation sowohl in den verschiedenen circulären Formen als auch in linearer Form verwendet werden können. Fremde genetische Information bedeutet DNA oder RNA, die Selbstreplizierend und infektiös ist oder die stabil ins Genom integriert ist.

Andere Plasmide, die bezüglich der vorgegebenen Fragestellung zu gleichen Resultaten führen, sind z.B. pCAP212 (Velten und Schell, ; Nucl. Acids Res. 13 (1985), 6981-6997) oder Derivate von pDH51, wie pEP9 (Pietrzak et al., Nucl. Acids Res. 14 (1986), 5857-5868).

Gewebekultur und Transformationsmethoden

Die Versuche wurden in der Regel mit ungebeiztem Sommerweizen-Samen durchgeführt. Es kann aber auch Samen anderer Getreide verwendet werden wie Winterweizen, Sommergerste, Wintergerste, Sommerroggen, Winterroggen, Triticale, Hafer, Mais, Reis. und Sorghum oder anderer Nutzpflanzen, wie Erbse, Bohne, Sojabohne, Psophocarpus.

Der Begriff "reifer Samen" oder kurz "Samen" beschreibt ein der Verbreitung und der Arterhaltung dienendes Organ der höheren Pflanze, das aus dem Keimling (Embryo), dem Nährgewebe (Endosperm) und einer mehr oder weniger festen Samenschale (Testa) besteht.

Die Isolierung von Embryonen durch mechanische Einwirkung erfolgt gemäß der Vorschrift von Johnston und Stern (Nature 179 (1957), 160 - 161) mit Ausnahme der Verwendung von Trockeneis: 650 g Weizensamen in 130 g Portionen wurden 10 Sekunden in einem 1 l Behälter bei geringster Geschwindigkeit mittels Waring Blendor® zerkleinert. Die zerschlagenen Körner wurden mit Prüfsieben (1,6 mm; 1,0 mm; 0,5 mm Maschenweite) fraktioniert. Die 1,6 mm Fraktion wurde dreimal in gleicher Weise behandelt. Kleie- oder Spelzenanteile wurden mit hilfe eines Gebläses entfernt und die Embryonen vom Endosperm durch Flotieren mit Cyclohexan/Tetrachlorkohlenstoff getrennt. Tabelle I gibt die empirisch bestimmten Mischungsverhältnisse von Cyclohexan mit Tetrachlorkohlenstoff zur Embryo-Endospermtrennung für die einzelnen Getreide wieder. Abschließend wurden mehr oder weniger vollständige Embryonen mittels Sieb (0,71 mm Maschenweite) von Embryobruchstücken abgetrennt. Diese 0,71 mm Fraktion ist in Figur 1b wiedergegeben. Die Ausbeute an Embryonen entspricht für Sommerweizen etwa 40 %.

Tabelle II

| Cyclohexan/Tetrachlorkohlenstoff Mischungsverhältnisse zur Trennung von Embryonen und Endosperm verschiedener Getreide | | | |
|---|---|---|---|
| Getreide | Anteile Cyclohexan | Anteile Tetrachlorkohlenstoff | Bemerkungen |
| So-Weizen | 100 | 250 | |
| Wi-Weizen | 100 | 250 | |
| So-Gerste | 100 | 250 | |
| Wi-Gerste | 100 | 250 | |
| Wi-Roggen. | 100 | 300 | |
| Triticale | 100 | 330 | |
| Hafer | 100 | 250 | |
| Mais | 100 | 200 | schlechte Trennung |
| Reis | 100 | 250 | |
| Sorghum | 100 | 200 | |

Die DNA-Behandlung von Weizenembryonen in ihrer optimierten Form gestaltete sich wie folgt: 150 mg isolierte Embryonen wurden 30 Minuten bei Raumtemperatur in 500 µl eines Inkubationspuffers aus
200 µg Plasmid DNA ( = 73 pM)
15 mM NaCl

1,5 mM Trinatriumcitrat
20 % DMSO
aufgefüllt mit Wasser auf einen Milliliter
inkubiert. Auf einem Filter auf festem MS Medium (Murashige und Skoog, Plant Physiol. 15 (1962), 473-497) ohne Hormone mit 100 µg Claforan ausplattiert, erfolgte die Kultur der Embryonen bei 24°C, 12 Std. Photoperiode und 600 lux. Nach etwa 3 Tagen Kultur wurden die Embryonen auf Genexpression untersucht.

### Versuche am Embryo im Samen

Neben isolierten Embryonen wurden auch Embryonen im Weizensamen mit DNA behandelt. Dazu wurde zunächst der Embryo durch vorsichtiges Abschleifen des Perikarps freigelegt und dann mit 2 bis 3 µl des vorstehenden Inkubationspuffers befeuchtet. Sofort oder nach dem Antrocknen wurden die so behandelten Samen zum Studium der transienten Expression entweder auf feuchtem Filterpapier kultiviert oder für die stabile Transformation auf Erde ausgelegt. Letztere wurden in Multitopfplatten (96 Töpfe) bei etwa 24°C im Gewächshaus 4 Tage mit einer Folie abgedeckt und anschließend mit Erde übersiebt.

### Enzymtest Verfahren

NPT-Test (Reiss et al., Gene 30 (1984), 211-218; Schreier et al., EMBO J. 4 (1985), 25-32; modifiziert)

Der NPT-Test wurde unter Verwendung von 100 µCi [$^{32}P_\gamma$ -ATP] pro Test und einigen Abwandlungen der Probenaufbereitungen durchgeführt: Die gekeimten Embryonen wurden in 2 ml Extraktionspuffer (62,5 mM Tris-HCl, pH 6,8 und 5 % $\beta$-Mercaptoäthanol) auf Eis in einem Mörser zerkleinert. Das Homogenat wurde anschließend 60 Minuten bei 100000 x g zentrifugiert und NPT II aus dem Überstand bei 50 % $(NH_4)_2SO_4$ gefällt (Zentrifugationen 9000 x g, 15 Min., 4°C). NPT II kann durch Ammoniumsulfat oberhalb 30 % ausgefällt werden. Bei einem Wert von etwa 45 % ist NPT II nicht mehr im Überstand zu finden. Das Proteinsediment wurde vorsichtig mit 250 µl Extraktionspuffer gewaschen und dann in 50 µl Puffer (62,5 mM Tris-HCl, pH 6,8; 5 % $\beta$-Mercaptoäthanol, 10 % Glycerin und 0,025 % Bromphenolblau) gelöst.
Die Beispiele erläutern die Erfindung.

### Beispiel 1

### Transiente Expression in isolierten Weizenembryonen

Erste, nur gelegentlich auftretende NPT II-Signale wurden nach der Inkubation von 15 mg trockenen Weizenembryonen mit 100 µg DNA pro Milliliter in 15 mM NaCl und 1,5 mM Trinatriumcitrat nach drei Tagen Kultur erhalten. Es galt zu beweisen, daß es sich nicht um ein Artefakt handelte:
In einer Versuchsreihe wurden unterschiedliche Mengen (15, 50, 100, 200, 300, 400 mg) an Embryonen in DNA-Lösung (100 µg Plasmid-DNA pro Milliliter, 15 mM NaCl, 1,5 mM Trinatriumcitrat) gequollen und anschließend kultiviert. Doppelproben sollten zeigen, ab welcher Embryomenge regelmäßig und reproduzierbar NPT II-Signale auftreten. Zwei Signale wurden bei 200, 300 und 400 mg eingesetzter Embryonen festgestellt. Die Signale bei nur 200 mg waren jedoch noch recht schwach, so daß für weitere Experimente zunächst 300 mg Embryonen eingesetzt wurden.
Figur 1a zeigt das Ergebnis eines Experimentes von DNA-behandelten Embryonen (Dreifachproben: Spur 3-5) im Vergleich zu extrahierten Embryonen ohne DNA-Behandlung (Spur 2).
Sodann wurde untersucht, ob trockene Embryonen für eine DNA-Aufnahme erforderlich sind. Ein einfaches Experiment sollte den Vorgang der DNA-Aufnahme zeigen. 300 mg Embryonen wurden wie bisher zunächst 30 Minuten mit DNA-Lösung behandelt, dann aber zusätzlich 30 Minuten mit der DNA-freien Lösung. Mit weiteren 300 mg Embryonen wurde umgekehrt verfahren. Zuerst wurden sie mit Lösung ohne DNA und dann mit einer Lösung mit DNA inkubiert. Das Ergebnis (Doppelproben) ist in Figur 2 wiedergegeben. Nur trockene Embryonen sind in der Lage, DNA aufzunehmen und nach Kultur zu exprimieren. Außerdem erfolgt die DNA-Aufnahme innerhalb der ersten 30 Min. der Quellung. Weitere Experimente haben gezeigt, daß die DNA-Aufnahme innerhalb der ersten drei Minuten an höchsten ist, dann langsam abfällt und nach 15 Minuten weitgehend abgeschlossen ist.
Anschließend wurde untersucht, wie sich die Expression steigern läßt. Bisher war unklar, ob die gewählten Anfangsbedingungen optimal waren. Die folgenden Versuche dienten der Klärung dieser Frage sowie einer generellen Verbesserung des Expressionssystems: Zunächst wurde DMSO getestet. Eine deutliche Steigerung der Expression mit zunehmender DMSO Konzentration bis 20 % wurde festgestellt (Fig. 3). Liegen die DMSO Konzentrationen bei 30 % oder höher, ist das Frischgewicht reduziert. Entsprechend sinkt die NPT II Expression. Nach diesem Ergebnis wurde die vorstehend beschriebene DNA-Lösung (100 $\mu$g Plasmid-DNA pro Milliliter, 15 mM NaCl, 1,5 mM Trinatriumcitrat) wie folgt modifiziert: 100 $\mu$g Plasmid-DNA pro Milliliter, 15 mM NaCl, 1,5 mM Trinatriumcitrat, 20 % DMSO. Eine erneute Versuchsreihe mit verschiedenen Embryomengen ergab eine Reproduzierbarkeit der NPT II-Signale bis 30 mg eingesetzter Embryonen und vergleichbar kräftige Signale bei 150 und 300 mg (Fig. 3, oben). Somit wurde die Embryomenge auf 150 mg reduziert.

Mit den beiden geänderten Parametern wurde nun nach der DNA Konzentration gesucht, die maximale NPT II-Expression erlaubt. Der Vergleich von 25, 50, 100 und 200 $\mu$g Plasmid pro 0,5 ml Inkubationsansatz (150 mg Embryonen, Plasmid-DNA, 15 mM NaCl, 1,5 mM Trinatriumcitrat, 20 % DMSO) zeigte die höchste Expression bei 100 und 200 $\mu$g DNA pro 0,5 ml (Fig. 3). Damit war unter Verwendung von 100 $\mu$g Plasmid DNA pro 0,5 ml (bzw. 200 $\mu$g pro ml) dieser Parameter ins Optimum gerückt.

Der optimale Inkubationsansatz bestand daher aus:

150 mg trockenen Weizenembryonen

+

100 $\mu$g Plasmid-DNA (36,5 mM)

15 mM NaCl

1,5 mM Trinatriumcitrat

20 % DMSO

aufgefüllt mit Wasser auf 0,5 Milliliter.

Inkubiert man Embryonen mit diesem Inkubationspuffer so ist NPT II bereits nach 24 Stunden Kultur zuverlässig nachweisbar. Bei einer Kultur von 24, 48 und 72 Stunden zeigt sich eine stetige Zunahme der NPT II-Aktivität.

Beispiel 2:

Transiente Expression in Weizenembryonen am Samen

Es wurden Weizensamen verwendet, bei denen der Embryo durch Anschleifen freigelegt worden war. Die Weizensamen wurden mit DNA (gemäß den vorstehenden optimierten Bedingungen) behandelt und 3 Tage angekeimt. Die Keimlinge wurden sodann isoliert und auf NPT II-Aktivität untersucht. Wie auch bei isolierten, behandelten Embryonen wurde NPT II nachgewiesen. Damit war gezeigt, daß auch einseitig behandelte Embryonen DNA aufnehmen und exprimieren.

Beispiel 3:

Transiente Expression in Getreideembryonen

Mit den für Sommerweizen-Embryonen etablierten Methoden konnte eine transiente Expression auch in Embryonen von Winterweizen, Sommergerste, Wintergerste, Roggen, Hafer, Mais, Reis und Triticale nachgewiesen werden.

Beispiel 4:

Transiente Expression in Leguminosenembryonen

300 mg Gartenerbsen-, Feuerbohnen- und Saubohnenembryonen wurden in Analogie zu Weizenembryonen mit DNA inkubiert. Nach drei Tagen Kultur konnte NPT II-Aktivität nachgewiesen werden.

Beispiel 5:

Der Weg ins Gewächshaus

Kultur von angeschliffenen Samen in Erde

10 Weizensamen wurden wie unter "Material und Methoden" beschrieben angeschliffen und mit 2 $\mu$l Wasser an der Schliffstelle behandelt. Sie wurden dann auf Einheitserde/Sand (10/1) (im weiteren kurz als "Erde" bezeichnet) zur Keimung ausgelegt. Als Kontrolle wurden unbehandelte Samen ausgelegt. Die Keimung des angeschliffenen Materials erfolgte schneller als die der Kontrollen. Nach 3 Tagen aber zeigten letztere eine bessere Entwicklung. Weitere Beobachtungen ergaben, daß die angeschliffenen Embryonen teilweise Verletzungen an Coleoptile, Coleorhiza und darunterliegenden Geweben aufwiesen. Auch das Ausbleiben einer Keimung, vermutlich aufgrund von Schädigungen, mußte festgestellt werden. Es wurden jedoch insgesamt 8 Keimlinge mit kräftigem Keimblatt von den angeschliffenen Samen erhalten.

Ein Anschlußversuch in Multitopfplatten mit je 96 DNA-behandelten, angeschliffenen Samen und 96 unbehandelten Kontrollen wurde unternommen. Nach drei Wochen Kultur der im Gewächshaus bei 24 °C auf Erde und mit Quarzsand abgedeckten Samen, waren 83 Kontrollpflanzen gekeimt, von den DNA-behandelten Pflanzen jedoch nur 21. Das bedeutet eine Erfolgsquote von etwa 23 % (Tabelle III).

In einem weiteren Experiment wurden je 288 angeschliffene und DNA-behandelte Samen etwa 1-2 cm tief in die Erde gelegt, auf Erde liegend mit Erde übersiebt bzw. nur auf Erde ausgelegt, vier Tage mit Kunststoffolie abgedeckt und dann übersiebt. Das Versuchsergebnis nach 3 Wochen geht ebenfalls aus Tabelle III hervor. Die höchste Keimrate wiesen die mit Folie abgedeckten Samen mit 63 % auf. Aufgrund dieses Resultats wurden in den folgenden Versuchen die angeschliffenen und behandelten Samen auf Erde ausgelegt und während der ersten vier Tage mit einer Folie abgedeckt. Danach wurden sie mit Erde übersiebt.

Tabelle III

| Einfluß der Kulturbedingungen auf die Keimrate von angeschliffenen Weizensamen. | | | |
|---|---|---|---|
| Anzahl ausgelegter Samen | Anzahl gekeimter Samen | Keimungsrate | variierter Parameter |
| 96 | 22 | 23 % | Quarzabdeckung |
| 96 | 83 | 86 % | Kontrolle, Quarzabdeckung |
| 288 | 49 | 17 % | 1 bis 2 cm in Erde |
| 288 | 71 | 25 % | auf erde, übersiebt |
| 288 | 181 | 63 % | auf erde, 4 Tage Folienabdeckung, dann Übersieben |

Von 11000 ausgelegten, angeschliffenen und DNA-behandelten Samen wurden ca. 4300 Pflanzen bis zur Samenreife herangezogen. Das geerntete Saatgut wurde unter Selektionsbedingungen, entsprechend dem Marker-Gen (z.B. Kanamycinresistenz durch NPT II) gekeimt, und die überlebenden Pflänzchen wurden sowohl auf das Genprodukt des Marker-Gens, als auch auf das Vorhandensein der zugegebenen DNA untersucht.

**Patentansprüche**

1. Freigelegte reife Embryonen von Nutzpflanzen mit einem mechanisch Wassergehalt von höchstens etwa 20 Gewichtsprozent als Aufnahmesystem für fremde genetische Information in Form von DNA oder RNA erhältlich durch Abschleifen der Samenschale und Verletzung des Embryos.

2. Freigelegte reife Embryonen von Nutzpflanzen nach Anspruch 1 mit einem Gehalt an eingeschleuster fremder genetischer Information in Form von DNA oder RNA.

3. Embryonen nach Anspruch 2, bei denen die fremde genetische Information in Form von DNA vorliegt und in das Genom integriert ist.

4. Embryonen nach Anspruch 2 oder 3, bei denen die fremde genetische Information in Form von DNA oder RNA vorliegt, die in der Pflanze Verbreitet, aber nicht in Gas Genom integriert ist.

5. Verfahren zur Herstellung von Embryonen nach Anspruch 1, bei dem man die Samenschale des den Embryo enthaltenden Samens im Bereich des Embryos, der einen Wassergehalt von höchstens etwa 20 Gewichtsprozent aufweist, so abschleift, daß der Embryo für eine fremde genetische Information in Form von DNA oder RNA zugänglich wird, jedoch in seiner Keimfähigkeit nicht wesentlich beeinträchtigt wird.

**6.** Verfahren zur Herstellung von Embryonen nach einem der Ansprüche 2 bis 4, bei dem man freigelegte reife Embryonen von Nutzpflanzen mit einer wäßrigen Lösung der genetischen Information in Form von DNA oder RNA behandelt.

**7.** Verfahren nach Anspruch 5 oder 6, dadurch gekennzeichnet, daß die wäßrige Lösung einen pH-Wert von 3 bis etwa 11 hat.

**8.** Verwendung der Embryonen nach einem der Ansprüche 2 bis 4 zur Regenerierung und Züchtung transgener Nutzpflanzen.

**Claims**

**1.** Mechanically exposed, mature embryos of useful plants having a water content of no more than about 20 weight percent as the receiving system for foreign genetic information in the form of DNA or RNA, obtainable by abrading the seed coat and injuring the embryo.

**2.** The exposed, mature embryos of useful plants according to claim 1 having a content of introduced foreign genetic information in the form of DNA or RNA.

**3.** The embryos according to claim 2, wherein the foreign genetic information is present in the form of DNA and is integrated in the genome.

**4.** The embryos according to claim 2 or 3, wherein the foreign genetic information is present in the form of DNA or RNA, which is spread in the plant but is not integrated in the genome.

**5.** A process for preparing embryos according to claim 1, wherein the coat of the embryo-containing seed is so abraded in the area of the embryo which has a water content of no more than about 20 weight percent, that the embryo is accessible to foreign genetic information in the form of DNA or RNA, but is not substantially impaired in its germinative abilities.

**6.** The process for preparing the embryos according to any of claims 2 to 4, wherein the exposed, mature embryos of useful plants are treated with an aqueous solution of the genetic information in the form of DNA or RNA.

**7.** The process according to claim 5 or 6, characterized in that the aqueous solution has a pH value of 3 to about 11.

**8.** The use of the embryos according to any one of claims 2 to 4 for regenerating and culturing transgenic useful plants.

**Revendications**

**1.** Embryons à maturité, mécaniquement libérés, de plantes utiles présentant une teneur en eau d'au maximum environ 20 % en poids, comme système de captage d'une information génétique étrangère sous la forme d'ADN ou d'ARN, que l'on peut obtenir par abrasion du tégument et lésion de l'embryon.

**2.** Embryons à maturité, mécaniquement libérés, de plantes utiles suivant la revendication 1, présentant une teneur en information génétique étrangère, éclusée, sous la forme d'ADN ou d'ARN.

**3.** Embryons suivant la revendication 2, dans lesquels l'information génétique étrangère se présente sous la forme d'ADN et est intégrée dans le génome.

**4.** Embryons suivant l'une des revendications 2 et 3, dans lesquels l'information génétique étrangère se trouve sous la forme d'ADN ou d'ARN qui se propage dans la plante, mais elle n'est pas intégrée dans le génome.

**5.** Procédé de préparation d'embryons suivant la revendication 1, dans lequel on soumet à une abrasion le tégument de la graine contenant l'embryon, dans la zone de l'embryon qui présente une teneur en eau d'au maximum environ 20 % en poids, de façon que l'embryon soit accessible pour une information génétique étrangère sous la forme d'ADN ou d'ARN, mais ne soit cependant pas influencé de manière sensiblement désavantageuse dans sa capacité de germination.

6. Procédé de préparation d'embryons suivant l'une des revendications 2 à 4, dans lequel on traite des embryons à maturité, libérés, de plantes utiles avec une solution aqueuse de l'information génétique sous la forme d'ADN ou d'ARN.

7. Procédé suivant l'une des revendications 5 et 6, caractérisé en ce que la solution aqueuse présente une valeur de pH de 3 à environ 11.

8. Utilisation des embryons suivant l'une des revendications 2 à 4, pour la régénération et la culture de plantes utiles transgéniques.

EP 0 362 244 B1

Fig. 1

14

Fig. 2

Fig. 3

# Fig. 4A

17

Fig. 4B

EP 0 362 244 B1

```
Hind3 SphI  PstI  HincII         .         .         .         .         .         .         .         .         .         .
aagcttgcatgcctgcaggtcAACATGGTGGAGCACGACACTCTCGTCTACTCCAAGAATATCAAAGATACAGTCTCAGAAGACCAGAGGGCTATTGAGACTTTTCAACAAAGGGTAATA 120
        .         .         .         .         .         .         .         .         .         .         .         .
TCGGGAAACCTCCTCGGATTCCATTGCCCAGCTATCTGTCACTTCATCGAAAGGACAGTAGAAAAGGAAGATGGCTTCTACAAATGCCATCATTGCGATAAAGGAAAGGCTATCGTTCAA 240
        .         .         .         .         .         .         .         .         .         .    EcoRV       .
GAATGCCTCTACCGACAGTGGTCCCAAAGATGGACCCCCACCCACGAGGAACATCGTGGAAAAAGAAGACGTTCCAACCACGTCTTCAAAGCAAGTGGATTGATGTGATATCTCCACTGA 360
        .         .         .         .         .         .         .    . XhoI ApaINcoI    SstI  KpnISmaI BamHI .
CGTAAGGGATGACGCACAATCCCACTATCCTTCGCAAGACCCTTCCTCTATATAAGGAAGTTCATTTCATTTGGAGAGGACctcgagggcccatgggcgagctcggtacccggggatcct 480
XbaI    .         .         .         .         .         .         .         .         .         .         .
ctagagtcCGCAAATCACCAGTCTCTCTCTACAAATCTATCTCTCTCTATTTTCTCCAGAATAATGTGTGAGTAGTTCCCAGATAAGGGAATTAGGGTTCTTATAGGGTTTCGCTCATGT 600
        .         .         .         .         .         .         .         .         .    PstI  SphI  Hind3
GTTGAGCATATAAGAAACCCTTAGTATGTATTTGTATTTGTAAAATACTTCTATCAATAAAATTTCTAATTCCTAAAACCAAAATCCAGTgacctgcaggcatgcaagctt 711
```

Fig. 5